# EUROPÄISCHE PATENTSCHRIFT

(11) **EP 0 867 160 B1**
(45) Veröffentlichungstag und Bekanntmachung des Hinweises auf die Patenterteilung: **31.07.2002**
(21) Anmeldenummer: 98103496.0
(22) Anmeldetag: 27.02.1998
(51) Int. Cl.: A61F 2/36, A61F 2/30

(54) **Hüftgelenkprothese**
Hip joint prosthesis
Prothèse de l'articulation de la hanche

(30) Priorität: 26.03.1997 DE 29705498 U
(43) Veröffentlichungstag der Anmeldung: 30.09.1998
(73) Patentinhaber: Waldemar Link (GmbH & Co.), 22339 Hamburg (DE)
(72) Erfinder: Keller, Arnold, 23863 Kayhude (DE)
(74) Vertreter: Glawe, Delfs, Moll & Partner

(56) Entgegenhaltungen:
- EP-A- 0 012 146
- EP-A- 0 093 378
- EP-A- 0 169 976
- EP-A- 0 295 360
- EP-A- 0 393 425
- FR-A- 1 432 116
- FR-A- 2 595 241
- FR-A- 2 602 672
- FR-A- 2 687 306
- US-A- 5 163 964
- US-A- 5 360 446

## Beschreibung

Die Erfindung betrifft eine Hüftgelenkprothese mit einem in den Oberschenkelknochen einzusetzenden Schaft, der an seinem oberen Ende mindestens auf der Vorder- und Rückseite einstückig mit einer Halsauflage verbunden ist und Längsnuten aufweist.

Längsnuten im Schaft verbessern die Kraftübertragung von der Prothese auf den umgebenden Knochen. Das ist vor allem bei zementloser Implantation wichtig. In diesem Fall wächst während des Heilungsprozesses Knochengewebe in die Nuten ein und verbindet sich mehr oder weniger fest mit der Prothesenoberfläche. Aber auch bei zementierter Implantation kann die Ausrüstung des Prothesenschafts mit Nuten zweckmäßig sein. Wenn im Falle einer Reoperation das in den Nuten befindliche Knochen- oder Zementmaterial wieder gelöst werden muß, muß der Operateur mit einem geeigneten Meißel von proximal her in die Nuten eindringen. Voraussetzung dafür ist die Zugänglichkeit der Nuten von ihrem proximalen Ende her.

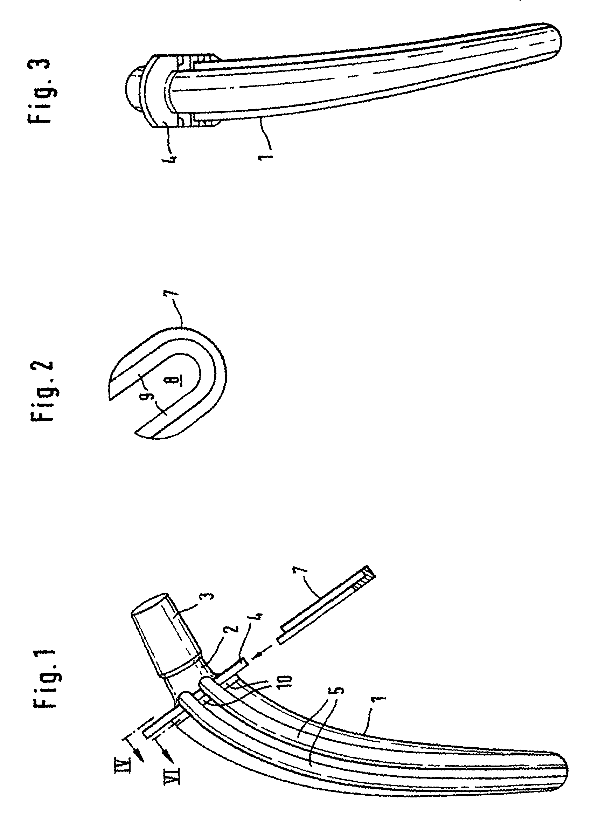

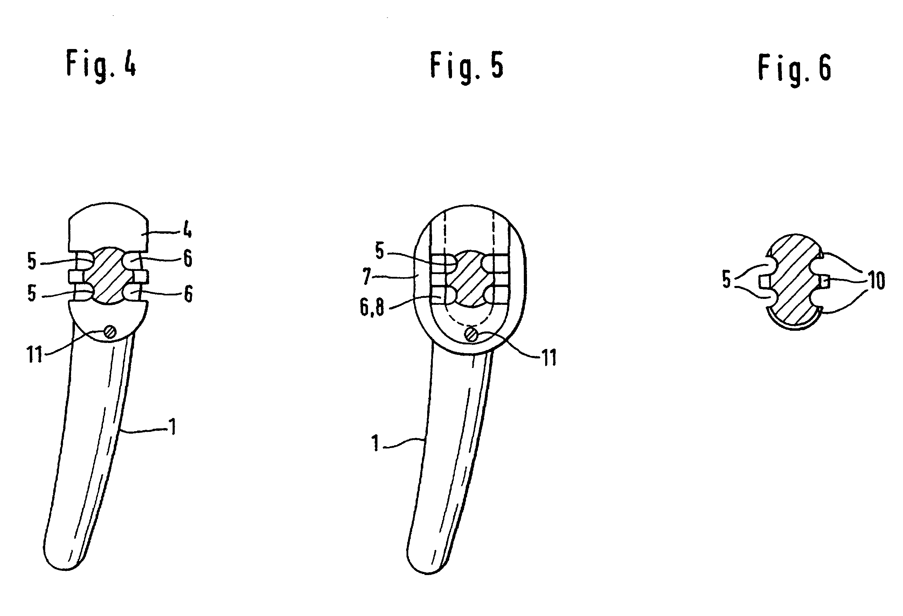

Es ist eine femorale Hüftgelenkprothese bekannt (EP-B 93378, Fig. 1 und 2), die eine einstückig mit dem Schaft ausgebildete Halsauflage aufweist. Der Schaft ist mit Längsrippen versehen, zwischen denen tiefe Nuten gebildet sind. Die Rippen und Nuten gehen am oberen Ende bis an die Halsauflage heran und werden von dieser begrenzt. Da sie von der Gelenkseite her den Nutraum abdeckt, ist dieser im Falle einer Reoperation schwer zugänglich. Es ist daher auch vorgeschlagen worden (a.a.O., Fig. 5), den Ansatzteil bei implantierter Prothese abnehmbar zu machen, ohne daß die Art der Verbindung mit der Prothese im einzelnen offenbart wird. Bei einer ähnlichen bekannten Prothese (Prospekt der Waldemar Link (GmbH & Co): "Link Rippensystem") sind die Schenkel der hufeisenförmig ausgebildeten Halsauflage in vorder- und rückseitigen Führungsnuten des Schafts gehalten. Wenn die Halsauflage abgenommen ist, sind im Bereich der Nuten keine vorspringenden Teile vorhanden, die den operativen Zugang hemmen könnten. Die quer zur Längsrichtung des Schafts verlaufenden Führungsnuten sind jedoch in manchen Fällen unerwünscht, weil sie den Querschnitt des Schafts und damit seine Festigkeit verringern. Auch ziehen manche Ärzte eine einstückig mit dem Schaft verbundene Halsauflage im Hinblick auf deren Zuverlässigkeit vor.

Es ist eine femorale Hüftgelenkprothese bekannt (FR-A 2 595 241), die diesem Problem dadurch aus dem Weg geht, daß lediglich an der medialen Schmalseite des Prothesenschafts, die keine Nuten enthält, ein kleiner Vorsprung in der Art einer Halsauflage vorgesehen ist, während die mit Nuten versehenen Vorder- und Rückseiten des Schafts keine Halsauflage besitzen. Jedoch ist man bestrebt, eine Halsauflage großflächig auszubilden, um die spezifische Belastung des Knochens durch die Halsauflage gering zu halten. Die Erfindung setzt daher voraus, daß die Halsauflage auch auf denjenigen Seiten des Schaftes vorhanden ist, die mit Nuten ausgerüstet sind.

Die Erfindung schafft eine Hüftgelenkprothese, deren Nuträume trotz Anwesenheit einer einstückig mit dem Schaft verbundenen Halsauflage zugänglich sind. Die Lösung besteht darin, daß die Halsauflage im Bereich der Nuten ausgeschnitten ist und die Längsnuten oberhalb der Halsauflage enden. Mit anderen Worten sind die Längsnuten im Bereich der Halsauflage durchgeführt, wobei diese von den Nuten durchschnitten wird. Es ist daher möglich, bei der Reoperation mit einem den Knochenverbund im Bereich der Nuten lösenden Werkzeug in diese einzugreifen oder bei der Implantation den Nutraum zum Nachstopfen von Knochenmaterial zu nutzen. Nichtsdestoweniger weist die Prothese eine einstückig mit dem Schaft verbundene Halsauflage auf. In vielen Fällen reicht die von der Halsauflage außerhalb der Ausschnitte gebotene Fläche zur Abstützung aus. Wenn eine größere Auflagefläche erwünscht ist, kann sie erfindungsgemäß mit einem die Ausschnitte im Bereich der Längsnuten im wesentlichen schließenden, lösbaren Ansatzteil versehen sein. Die Halsauflage und/oder der Schaft bilden eine parallel zur Erstreckung der Halsauflage verlaufende Schiebeführung, längs welcher der Ansatzteil bei implantierter Prothese einsetzbar bzw. entfernbar ist.

Die Erfindung wird im folgenden näher unter Bezugnahme auf die Zeichnung erläutert, die ein vorteilhaftes Ausführungsbeispiel veranschaulicht. Darin zeigen:
- Fig. 1: die Prothese in Seitenansicht mit dem aufzuschieben den Ansatzteil in Schnittansicht,
- Fig. 2: den aufzuschiebenden Ansatzteil in Draufsicht,
- Fig. 3: eine Lateralansicht der Prothese,
- Fig. 4: eine Schnittansicht gemäß Linie IV der Fig. 1,
- Fig. 5: eine der Fig. 4 entsprechende Ansicht mit eingesetztem Ansatzteil und
- Fig. 6: einen Schnitt gemäß Linie VI der Fig. 1.

Der Prothesenschaft 1, der in den Hüftknochen einzusetzen ist, geht am oberen Ende in einen Hals 2 über, der einen Konus 3 zur Anbringung eines Gelenkkopfs trägt. Zwischen Schaft 1 und Hals 2 ist eine Halsauflage 4 angeordnet, die nach allen Seiten, mindestens aber auf der Vorder- und Rückseite vom Schaft bzw. Hals seitlich wegsteht und eine ebene Unterfläche aufweist, die zur Auflage auf einer Resektionsfläche des Knochens bestimmt ist.

Der Schaft 1 enthält auf der Vorder- und Rückseite je zwei Nuten 5, die parallel zur Mittellinie des Schafts verlaufen. Sie dienen, wie bekannt, der innigeren Verbindung der Prothesenoberfläche mit dem umgebenden Knochenmaterial, das an dieser Stelle bei der Herstellung der den Prothesenschaft aufnehmenden Höhlung stehengelassen wird oder bei der Verdrängung von Knochenmaterial durch den Prothesenschaft dorthin gelangt oder durch Nachstopfen in diesem Bereich verdichtet wird oder anschließend hineinwächst. Die Querschnittsgestalt des Schaftes und der Nuten ist aus Fig. 6 ersichtlich und zwar aus deren äußerer Kontur.

Damit der Nutquerschnitt bei der Implantation zum Nachstopfen von Knochenmaterial oder bei der Explantation für die Lösung des Verbunds zwischen Knochen und Prothesenoberfläche zugänglich ist, ist die Halsauflage 4 jeweils im Bereich der Nuten bei 6 ausgeschnitten, wie Fig. 4 zeigt. Man erkennt, daß trotzdem noch eine erhebliche Fläche verbleibt, die zur Abstützung beispielsweise auf einer Resektionsfläche je nach Lage des Falles genügen kann.

Wenn die verbleibende Fläche der Halsauflage 4 nicht ausreicht, fügt man den Ansatzteil 7 hinzu. Dieser hufeisenförmige Teil weist seinem Innenraum 8 benachbarte, parallele Führungsränder 9 auf. Diesen entspricht unterhalb der Halsauflage 4 am oberen Ende des Prothesenschafts 1 auf der Vorund Rückseite je eine Aufnahmenut 10. Wird der Ansatzteil 7 in der in Fig. 1 gezeigten Pfeilrichtung auf den Schaft aufgeschoben, so werden seine Führungsränder 9 von den Nuten 10 aufgenommen und gehalten. Mittels einer Schraube 11 kann er an Ort und Stelle arretiert werden. Wie man in Fig. 5 erkennt, vergrößert der Ansatzteil 7 nicht nur die Auflagefläche im vorderen, hinteren und medialen Bereich, sondern schließt er auch großenteils die Ausschnitte 6. Es ist nicht erforderlich, sie vollständig auch nahe dem Schaftkern zu schließen, da die Stützwirkung sich hauptsächlich im kortikalen, außenliegenden Knochenbereich entfaltet.

Aus der Bezeichnung des mit dem Schaft einstückig ausgebildeten Teils 4 als Halsauflage soll nicht geschlossen werden, daß diese alleine - d.h. ohne Ansatzteil 7 - die Funktion der Halsauflage erfüllen können muß. Vielmehr kann dieser Teil auch auf die Funktion der Befestigung des Ansatzteils beschränkt sein, so daß nur beide zusammen die Halsauflage bilden.

Die Nuten 5 sind, wie Fig. 1 zeigt, bis oberhalb der Halsauflage 4 geführt, wo sie sanft auslaufen. Auch dadurch wird das Ansetzen eines Werkzeugs erleichtert. Jedoch ist es auch denkbar, sie erst im Bereich der Halsauflage 4 oder unmittelbar darunter beginnen zu lassen.

## Patentansprüche

1. Hüftgelenkprothese mit einem in den Oberschenkelknochen einzusetzenden Schaft (1), der an seinem oberen Ende mindestens auf der Vorder- und Rückseite einstückig mit einer Halsauflage (4) verbunden ist und Längsnuten (5) aufweist, **dadurch gekennzeichnet, daß** die Halsauflage (4) im Bereich der Längsnuten (5) ausgeschnitten ist.

2. Prothese nach Anspruch 1, **dadurch gekennzeichnet, daß** die Längsnuten (5) oberhalb der Halsauflage (4) enden.

3. Prothese nach Anspruch 1 oder 2, **dadurch gekennzeichnet, daß** die Halsauflage (4) mit einem ihre Ausschnitte (6) im Bereich der Längsnuten (5) im wesentlichen schließenden, lösbaren Ansatzteil (7) versehen ist.

4. Prothese nach Anspruch 3, **dadurch gekennzeichnet, daß** die Halsauflage (4) und/oder der Schaft (1) eine parallel zur Erstreckung der Halsauflage (4) verlaufende Schiebeführung (10) bilden, längs welcher der Ansatzteil (7) bei implantierter Prothese einsetzbar bzw. entfernbar ist.

5. Prothese nach Anspruch 4, **dadurch gekennzeichnet, daß** die Halsauflage (4) und der Ansatzteil (7) oder der Schaft (1) zusammenwirkende Arretierungsmittel (11) aufweisen.

## Claims

1. A hip joint prosthesis with a shaft (1) to be inserted into the femur, which at its upper end is connected at least on the front side and the rear side in one piece with a neck support (4) and has longitudinal grooves (5), **characterised in that** the neck support (4) is cut out in the vicinity of the longitudinal grooves (5).

2. A prosthesis according to Claim 1, **characterised in that** the longitudinal grooves (5) end above the neck support (4).

3. A prosthesis according to Claim 1 or 2, **characterised in that** the neck support (4) is provided with a detachable adapter part (7) substantially closing its cut-outs (6) in the vicinity of the longitudinal grooves (5).

4. A prosthesis according to Claim 3, **characterised in that** the neck support (4) and/or the shaft (1) form a sliding guide (10) extending parallel to the extension of the neck support (4), along which sliding guide the adapter part (7) can be inserted or removed when the prosthesis is implanted.

5. A prosthesis according to Claim 4, **characterised in that** the neck support (4) and the adapter part (7) or the shaft (1) have co-operating locking means (11).

## Revendications

1. Prothèse d'articulation de la hanche, comportant une tige (1) à insérer dans l'os du fémur, qui est reliée d'un seul tenant à son extrémité supérieure, au moins sur la face avant et la face arrière, à un support formant col (4) et présente des rainures longitudinales (5), **caractérisée en ce que** le support formant col (4) est découpé dans la zone des rainures longitudinales (5).

2. Prothèse selon la revendication 1, **caractérisée en ce que** les rainures longitudinales (5) se terminent au-dessus du support formant col (4).

3. Prothèse selon la revendication 1 ou 2, **caractérisée en ce que** le support formant col (4) est pourvu d'une pièce rapportée (7) amovible, fermant sensiblement les entailles (6) du col dans la zone des rainures longitudinales (5).

4. Prothèse selon la revendication 3, **caractérisée en ce que** le support formant col (4) et/ou la tige (1) forment un guide coulissant (10) s'étendant parallèlement à l'étendue du support formant col (4), le long duquel la pièce rapportée (7) peut être insérée ou enlevée lorsque la prothèse est implantée.

5. Prothèse selon la revendication 4, **caractérisée en ce que** le support formant col (4) et la pièce rapportée (7) ou la tige (1) comportent des moyens d'arrêt (11) coopérant.
